(19)

Europäisches
Patentamt
European
Patent Office
Office européen
des brevets

(11) **EP 2 725 509 A1**

(12) **EUROPEAN PATENT APPLICATION**

(43) Date of publication:
**30.04.2014 Bulletin 2014/18**

(51) Int Cl.:
***G06F 19/22*** *(2011.01)*

(21) Application number: **13157122.6**

(22) Date of filing: **28.02.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **29.10.2012 KR 20120120649**

(71) Applicants:
• **Samsung SDS Co. Ltd.**
**Seoul 135-918 (KR)**
• **Industry-Academic Cooperation Foundation,**
**Yonsei University**
**Seoul 120-749 (KR)**

(72) Inventors:
• **Park, Min-Seo**
**Seoul 135-284 (KR)**
• **Park, Sang-Hyun**
**Seoul 120-749 (KR)**
• **Yeu, Yun-Ku**
**Seoul 120-749 (KR)**

(74) Representative: **Grünecker, Kinkeldey,**
**Stockmair & Schwanhäusser**
**Leopoldstrasse 4**
**80802 München (DE)**

(54) **System and method for aligning genome sequence**

(57)　　　There is Provided a method and system for aligning a genome sequence. The system for aligning a genome sequence includes a fragment sequence producing unit producing a plurality of fragment sequences from a read sequence, a filtering unit forming a set of candidate fragment sequences only including those of the plurality of the produced fragment sequences matching a target sequence, a fragment sequence elongating unit calculating the number of mapping positions of each of the candidate fragment sequences to the target sequence, selecting a fragment sequence in which the calculated number of mapping positions is higher than a predetermined value and elongating the selected fragment sequence until the number of mapping positions to the target sequence approaches the predetermined value or less, a mapping length calculating unit dividing the target sequence into a plurality of sections and calculating a total mapping length of the candidate fragment sequences by sections, and an aligning unit selecting a section in which the calculated total mapping length is a reference value or more and performing global alignment of the read sequence with respect to the selected section.

[FIG. 1]

**Description**

**1. Field of the Invention**

The present disclosure relates to a technique of analyzing a genome sequence.

**2. Discussion of Related Art**

The conventional Sanger sequencing is rapidly being substituted with next generation sequencing (NGS) producing a large volume of short sequences because of its low cost and fast data production. In addition, various NGS resequencing programs aimed at accuracy have been developed. However, recently, as NGS techniques are developed, a cost of producing a fragment sequence has decreased to half or less of the previous cost, and thus a large volume of data can be used. For this reason, a technique of accurately processing a large volume of short sequences in a short time is required.

A first step of the sequence recombination is mapping reads at exact positions of a reference sequence through a genome sequence alignment algorithm. Here, the problem is that there is a difference in genome sequence even in the same species due to various genetic variations. Due to an error made in sequencing, there may also be a difference in genome sequence. Accordingly, the genome sequence alignment algorithm necessarily has high mapping accuracy effectively in consideration of such difference and variations.

Consequently, to analyze genome information, it is necessary to have as much accurate data of total genome information as possible. To this end, most of all, the development of a genome sequence alignment algorithm with high accuracy and large throughput is required prior to the analysis of genome information. However, the conventional methods had limits in satisfying these requirements.

SUMMARY OF THE INVENTION

One or more exemplary embodiments provide a means for aligning a genome sequence which may ensure mapping accuracy, improve complexity in mapping and thus increase a processing speed.

According to an aspect of an exemplary embodiment, there is provided a system for aligning a genome sequence to align a read sequence to a target sequence. The system for aligning a genome sequence includes a fragment sequence producing unit that produces a plurality of fragment sequences from a read sequence, a filtering unit that forms a set of candidate fragment sequences from the plurality of the produced fragment sequences, a fragment sequence elongating unit that, calculates a number of mapping positions that map each of the candidate fragment sequences to the target sequence, selects a fragment sequence in which the calculated number of mapping positions exceeds a predetermined value, and elongates the selected fragment sequence until the number of mapping positions that map the selected fragment sequence to the target sequence approaches the predetermined value or is less that the predetermined value, a mapping length calculating unit that divides the target sequence into a plurality of sections and that calculates a total mapping length of the candidate fragment sequences for each of said plurality of sections, and an aligning unit that selects one of the plurality of sections for which the calculated total mapping length is equal to or greater than a reference value and that performs a global alignment of the read sequence with respect to the selected one of the plurality of sections. According to an aspect of another exemplary embodiment, there is provided a method of aligning a read sequence to a target sequence. The method includes producing a plurality of fragment sequences from the read sequence, forming a set of candidate fragment sequences from the plurality of the produced fragment sequences, calculating the number of mapping positions that map each of the candidate fragment sequences to the target sequence, selecting a fragment sequence in which the calculated number of mapping positions exceeds a predetermined number, elongating the selected fragment sequence until the number of mapping positions that map the selected fragment sequence to the target sequence approaches the predetermined value or is less than the predetermined value, dividing the target sequence into a plurality of sections and calculating a total mapping length of the candidate fragment sequences for each of said plurality of sections , and selecting one of the plurality of sections for which the calculated total mapping length is equal to or greater than a reference value , and performing a global alignment of the read sequence with respect to the selected one of the plurality of sections.

According to an aspect of another exemplary embodiment, there provided a system for aligning a genome sequence to align a read sequence to a target sequence. The system for aligning a genome sequence includes a fragment sequence producing unit that produces a plurality of fragment sequences from a read sequence, a filtering unit that forms a set of candidate fragment sequences from the produced fragment sequences matching a target sequence, a mapping length calculating unit that divides the target sequence into a plurality of sections and that calculates a total mapping length of the candidate fragment sequences for each of said plurality of sections, and an aligning unit that selects one of the plurality of sections in which the calculated total mapping length is equal to or greater than a reference value and that performs a global alignment of the read sequence with respect to the selected one of the plurality of sections.

BRIEF DESCRIPTION OF THE DRAWINGS

The above and other objects, features and advantages will become more apparent to those of ordinary skill in the art by describing in detail exemplary embodiments thereof with reference to the adhered drawings, in which:

FIG. 1 is a flowchart explaining a method of aligning a genome sequence 100 according to an exemplary embodiment;

FIG. 2 is a diagram explaining a process of calculating the minimum error bound (mEB) in step 108 of the method of aligning a genome sequence 100 according to the exemplary embodiment;

FIG. 3 is a diagram explaining a process for producing a fragment sequence in step 112 of the method of aligning a genome sequence 100 according to the exemplary embodiment;

FIG. 4 is a diagram explaining a process for selecting a mapping target section using a mapping histogram according to an exemplary embodiment;

FIG. 5 is a diagram explaining a method of reducing cycles of unnecessary global alignment during global alignment according to an exemplary embodiment; and

FIG. 6 is a block diagram of a system for aligning a genome sequence 600 according to an exemplary embodiment.

DETAILED DESCRIPTION OF EXEMPLARY EMBODIMENTS

Hereinafter, exemplary embodiments will be described in detail. However, the following embodiments are described in order to enable those of ordinary skill in the art to embody and practice the exemplary embodiments.

When it is determined that detailed descriptions of known art relating to the exemplary embodiments will unnecessarily obscure the point of the exemplary embodiments, the detailed descriptions will be omitted. In addition, the terms used below are defined in consideration of functions of elements used in the exemplary embodiments, which may be changed according to a purpose of a user, operator or practice. Therefore, the definitions of the elements should be made throughout the specification.

The technical spirit of the exemplary embodiments is determined by the claims, and the following Examples are only provided to effectively explain the technical spirit of the exemplary embodiments to one of ordinary skill in the art.

Prior to describing exemplary embodiments in detail, terms to be used herein will be defined.

First, the term "read sequence" (or abbreviated as "read") is a short-length genome sequence data output from a genome sequencer. A length of the read sequence varies generally from 35 to 500 base pairs (bp) depending on a kind of the genome sequencer, and generally expressed as four letters, A, C, G and T in the case of DNA.

The term "target genome sequence" refers to a reference sequence used to produce a total genome sequence from read sequences. In genome sequence analysis, a total genome sequence is completed by mapping large amounts of reads output from a genome sequencer with reference to a target genome sequence. In the exemplary embodiment, the target genome sequence may be a sequence predetermined in the genome sequence analysis (i.e., a total genome sequence of a human), or a genome sequence produced from the genome sequencer.

The term "base" is the minimum unit constituting a target genome sequence and a read. As described above, DNA is expressed with four letters such as A, C, G and T, each of which is called a base, and so is the read sequence.

The term "fragment sequence" (or "seed") is a sequence serving as a unit in comparison of a read sequence with a target genome sequence to map the read sequence. Theoretically, to map the reads to the target genome sequence, all of the reads are sequentially compared with the target genome sequence from the very beginning, and mapping positions of the reads should be calculated. However, in this method, much time and computing power are required to map one read. Therefore, in practice, a piece formed of a part of the read, that is, a fragment sequence, is previously mapped to the target genome sequence, thereby finding mapping candidate positions of the total read sequences, and performing global alignment of the total read sequences to the corresponding candidate positions.

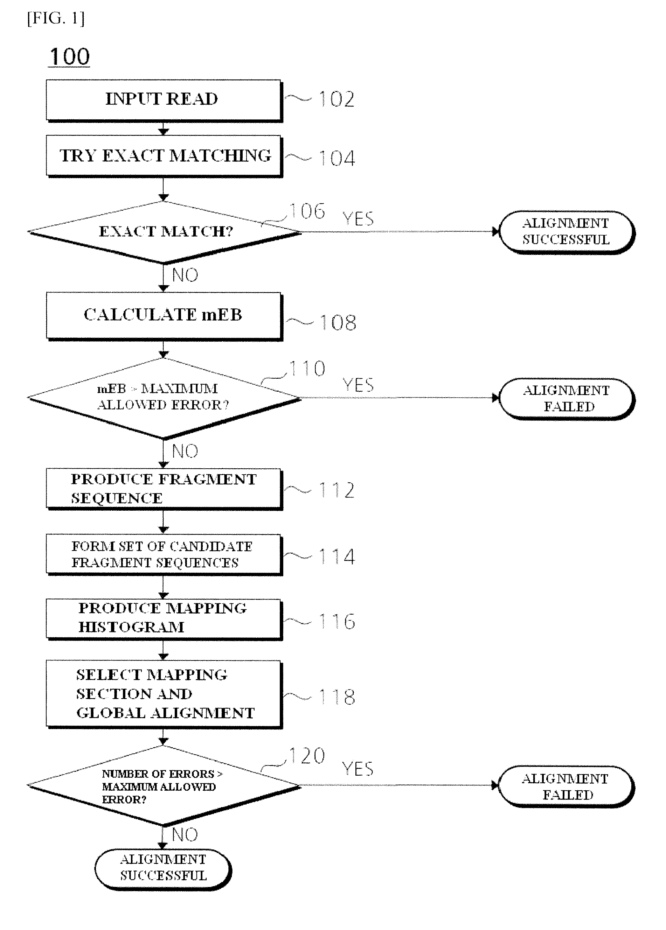

FIG. 1 is a flowchart explaining a method of aligning a genome sequence 100 according to an exemplary embodiment. In the exemplary embodiment, the method of aligning a genome sequence 100 refers to a series of processes for determining mapping (or aligning) positions of read sequences to a target sequence by comparing the read sequence output from a genome sequencer with the target genome sequence.

First, when the read sequences are input from the genome sequencer (step 102), exact matching with the target genome sequence is tried for all of the read sequences (step 104). As a result, if the exact matching with respect to all of the reads is successful, it is determined that aligning has succeeded without a subsequent step of alignment (step 106).

A test for a human genome sequence shows that 231,564 cycles of exact matching are performed among a total of 2,000,000 cycles of alignment (1,000,000 cycles for a forward sequence and 1,000,000 cycles for a reverse complement sequence) when 1,000,000 read sequences output from the genome sequencer are used in exact matching with the human genome sequence. Accordingly, after step 104, alignment consumption can be reduced by approximately 11.6%.

In contrast, when it is determined that the corresponding read sequence does not exactly match in step 106, mEB which may be shown in aligning of the corresponding read sequence to the target sequence is calculated (step 108).

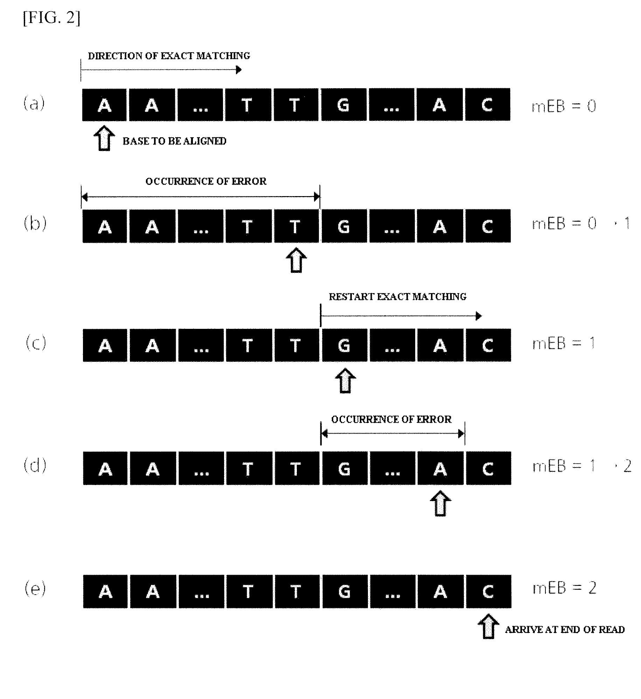

FIG. 2 is a diagram explaining a process of calculating mEB in step 108. First, as shown in FIG. 2 (a), the initial mEB is set to 0, and exact matching is tried base by base to the right from the very first base of the read sequence. Here, as shown in FIG. 2(b), it is assumed that exact matching is impossible from a specific base (the part represented as the second T in the drawing) of the read sequence. This means that there is an error somewhere in the section between a matching starting position and the current position of the read sequence. Therefore, in this case, the mEB value is raised by 1 (mEB=1), another exact matching starts from a next position (represented as (c) in FIG. 2). Afterward, if it is determined again that exact matching is impossible, another error has occurred somewhere in the section between the position at which the exact matching restarts and the current position. Therefore, the mEB value is raised again by 1 (mEB=2), and another exact matching starts from a next position (represented as (d) in FIG. 2). An mEB value obtained after exact matching approaches the end of the read throughout the process described above becomes an mEB value of the corresponding read.

When the mEB value of the read sequence is calculated throughout the process described above, it is determined whether or not the calculated mEB value exceeds the predetermined maximum allowed error (maxError) (step 110). If the mEB value exceeds the maxError, it is determined that the alignment with respect to the corresponding read sequence has failed, and then the alignment is terminated.

In the test for the human genome sequence described above, as the result of setting the maxError to 3 and calculating mEBs of the other reads, it is seen that reads corresponding to a total of 844,891 cycles of exact matching have values exceeding the maxError. That is, after step 108, the alignment consumption may be reduced by approximately 42.2%.

In contrast, in step 110, when it is determined that a calculated mEB value is the maxError or less, alignment of a corresponding read sequence is performed throughout the process to be described below.

First, a plurality of fragment sequences are produced from the read sequence (step 112), and a set of candidate fragment sequences including only those of the plurality of the produced fragment sequences matching the target sequence is formed (step 114). Afterward, the target sequence is divided into a plurality of sections, and a total mapping length of the candidate fragment sequences is calculated by sections, thereby producing a mapping histogram (step 116). In the produced mapping histogram, a section in which the total mapping length is a histogram cut or more is selected, and global alignment for the read sequences is performed in the selected section (step 118). Here, after the global alignment, when the number of errors of the read exceeds the predetermined maxError, it is determined that the alignment has failed, and otherwise it is determined that the alignment has succeeded (step 120).

Hereinafter, a specific process from steps 112 to 118 will be described in detail.

Production of Plurality of Fragment Sequences From Read sequence (Step 112)

This step is to produce a plurality of small pieces, that is, fragment sequences, from a read sequence to perform full-scale alignment of read sequences. In this step, the fragment sequences are produced by reading out values of a read sequence by a fragment size from the first base to the last base of the read sequence base by base up to a shift size.

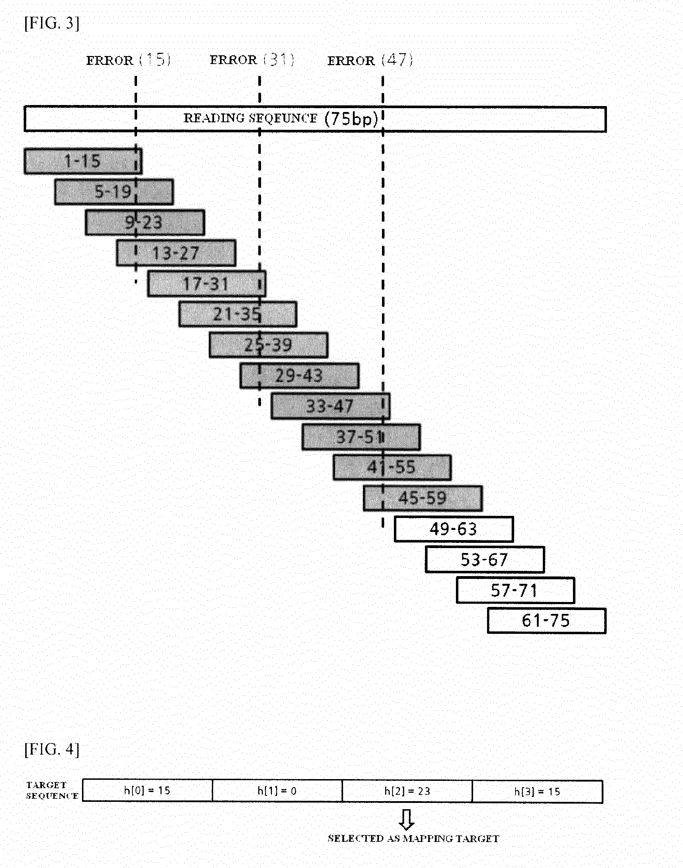

FIG. 3 is a diagram explaining a process of producing fragment sequences in step 112. In the illustrated exemplary embodiment, a length of a read sequence is 75 bp, the maxError of the read is 3 bp, a fragment size is 15 bp, and a shift size is 4 bp. That is, a fragment sequence is produced by 4 bp from the first base of the read sequence to the right. However, in the illustrated exemplary embodiment, the shift size and the fragment size may be suitably determined in consideration of values of a length of the read sequence and the maximum allowed error of the read. In other words, it is obvious that the scope of the inventive concept is not limited to specific fragment size and shift size.

Filtration and Elongation of Produced Fragment Sequence (Step 114)

After the fragment sequences are produced throughout the above-described process, a set of candidate fragment sequences is formed through a filtering process excluding those of the produced fragment sequences, which are not matching the target sequence. That is, the set of the candidate fragment sequences is formed from candidate fragment sequences in which the number of unmatched bases is a predetermined allowed value or less after exact matching of the produced fragment sequences with the target sequence. Here, when the allowed value is 0, the set of the candidate fragment sequences includes only fragment sequences exactly matching the target sequence.

For example, in the exemplary embodiment shown in FIG. 3, it is assumed that there are errors at the 15th, 31 st and 47th positions of the read (represented with a dotted line in FIG. 3). In this case, fragment sequences including the errors (represented in gray in FIG. 3) do not exactly match the target sequence, and only four of 49-63, 53-67, 57-71 and 61-75 fragment sequences, which are not affected by errors, exactly match the target sequence. Therefore, in this case, the set of the candidate fragment sequences includes only four fragment sequences described above.

Meanwhile, the target genome sequence (e.g., a human genome) generally includes several repeated sequences. Since these repeated sequences are distributed at several positions of the target sequence and repeatedly include the same genome sequence, some fragment sequences exactly match at too many positions in mapping with the target sequence. However, when too much mapping occurs in some fragment sequences due to the repeated sequences, it has a bad influence on complexity and accuracy of an entire alignment algorithm. Therefore, it is necessary to reduce the repetition of the mapping positions using a suitable method.

To this end, this step may further include elongating a size of a corresponding fragment sequence until the number of mapping positions is a predetermined value or less when the number of mapping positions of the candidate fragment sequences to the target sequence is more than the predetermined value (e.g., 50).

Particularly, in this step, the number of mapping positions of each of the candidate fragment sequences to the target sequence is calculated, fragment sequences having the calculated number of mapping positions exceeding the predetermined value are selected, and the size of the selected fragment sequence is elongated until the number of mapping positions to the target sequence is the predetermined value or less. Here, the elongation of the size of the selected fragment sequence may be performed by adding a base of the read sequence corresponding to a position of the selected fragment sequence from the beginning or end or end of the selected fragment sequence.

This will be described in detail. For example, it is assumed that a fragment sequence is produced from a read sequence as follows:

Read sequence: A T T G C C T C A G T

Fragment Sequence: T T G C (the underlined part of the read sequence)

If, after mapping for the fragment sequence, the number of mapping positions to the target sequence is 65, which is more than a reference value, 50, a length of the fragment sequence is elongated by 1 bp until the number of mapping positions is decreased to the reference value or less as shown below:

T T G C (65 mapping positions)

T T G C **C** (54 mapping positions)

T T G C **C T** (27 mapping positions)

In this case, since the number of mapping positions is decreased to the predetermined value or less when 2 bases are added with reference to the read sequence, a final fragment sequence becomes T T G C C T, which is elongated 2 bp longer than the initial value. Meanwhile, as described above in a different example, it should be understood that the scope of the inventive concept is not limited to a specific predetermined value because the predetermined value is also suitably determined according to characteristics of the target sequence, read sequence and fragment sequence.

In the test for a human genome sequence, when fragment sequences are produced to have a fragment size of 15 bp and a shift size of 4 bp from 1,000,000 reads and then matched to a target sequence, it is assumed that a reference value is 50, approximately 77% of 15,547,856 fragment sequences have 50 or fewer mapping positions. That is, it is assumed that the reference value is 50, the test result shows that 77% of the fragment sequences may be used without elongation, but the remaining 23% of the fragment sequences need fragment elongation according to the above-described method.

Production of Mapping Histogram (Step 116)

When a set of candidate fragment sequences (sub-candidates) is formed by the above-described process, it is basically possible to map a read sequence to a target sequence using mapping positions of the set of candidate fragment sequences in the target sequence. However, in this case, since all of the combinations of mapping positions of the candidate fragment sequences should be considered, complexity of calculation for mapping the read sequence is very high. For example, when 4 candidate fragment sequences are included in the set of candidate fragment sequences and the numbers of mapping positions of the respective candidate fragment sequences in the target sequence are 3, 6, 24 and 49, 21,168 (=3 x 6 x 24 x 49) combinations should all be examined. In the exemplary embodiment, to reduce the complexity of the calculation, a mapping histogram is used.

In the exemplary embodiment, the mapping histogram is an integer array having a regular size, and each of a plurality of sections of the target sequence is divided to have the same size. For example, when the target sequence is divided into sections having a size of 65,536 (=$2^{16}$) bp, a section of the target sequence from 0 to 65,535 bp corresponds to the first value of the mapping histogram (h), which is h[0], and a section of 65,536 to 131,071 corresponds to the second value of the mapping histogram (h), which is h[1]. In this manner, each section of the target sequence may correspond to the mapping histogram.

In addition, each value (h[i]) of the mapping histogram (h) stores a total mapping length (A) of the candidate fragment sequence in the target sequence section, and further stores the largest value (B) of positions of a candidate fragment sequence mapped to a corresponding target sequence section in the read sequence.

For example, in the exemplary embodiment shown in FIG. 3, when the 53-67 fragment sequence is mapped to the h[0] section, a histogram value of h[0] is (15, 67) (here, a total mapping length of the candidate fragment sequence mapped to the h[0] section is 15, and the final position of the mapped candidate fragment sequence in the read sequence is 67). In the same manner, when the 49-63 fragment sequence is mapped to the h[1] section, a histogram value of h[1] is (15, 63). Afterward, when the 61-75 fragment sequence is mapped to the h[0] section, a histogram value of h[0] is updated to (23, 75) for the following reason:

First Value 23: total mapping length considering the section in which the first-mapped 53-67 fragment sequence and the subsequently-mapped 61-75 fragment sequence overlap

Second Value 75: the final position of the mapped fragment sequences, corresponding to the final position of the 61-75 fragment sequence

Selection of Mapping Target Section and Global Alignment (Step 118)

When the mapping histogram is formed throughout the above-described process, a section in which a histogram value (total mapping length of candidate fragment sequences in the corresponding section) of the mapping histogram is a predetermined reference value (histogram cut) or more is selected as a mapping target section.

FIG. 4 is a diagram explaining a process of selecting a mapping target section using a mapping histogram according to an exemplary embodiment. As shown in FIG. 4, when a target sequence is divided into 4 sections from h[0] to h[3], a histogram value of each section is calculated as follows:

h[0] = 15, h[1] = 0, h[2] = 23, h[3] = 15

Here, when the histogram cut is set to 22, a section having a value larger than the histogram cut is the section corresponding to h[2], which is selected as a mapping target section in this step. Here, if there are a plurality of sections having a histogram value larger than the histogram cut, all of the corresponding sections become mapping target sections, and global alignment is performed in all sections included in the mapping target section. In this case, histogram values of the sections included in the mapping target section are compared with each other to raise an alignment speed, and global alignment may be sequentially performed from a section having a higher histogram value. The high histogram value means a long total length of the mapped fragment sequence, which is caused by high possibility of mapping of a read sequence in a corresponding section. In addition, when a histogram value of each section is the same, a number of candidate fragment sequences mapped to each section may be calculated, and global alignment may be performed from a section having the higher number of fragment sequences.

As described above, after the mapping target section is selected, those mapped to the corresponding mapping target section of the set of candidate fragment sequences (sub-candidates) are selected as final candidate fragment sequences (candidates), and then global alignment of the read sequence at each of the mapping positions of the selected final candidate fragment sequences is performed, thereby completing the alignment of the read sequence.

For example, when the candidate fragment sequences mapped to the h[2] section in the exemplary embodiment shown in FIG. 4 are 3, which are 49-63, 53-67 and 61-75 fragment sequences, the three candidate fragment sequences become final candidates, and global alignment of the read sequence may be performed at the mapping positions in the corresponding section.

Meanwhile, during the global alignment with respect to the final candidate fragment sequence, to reduce the time used in the global alignment, a position in the target sequence which has been subjected to the global alignment is remembered, thereby repeating global alignment at closer positions. Particularly, in this step, the mapping target section is divided into a plurality of sub-sections, and when global alignment is performed on a sub-section, the sub-section is recorded. Afterward, during global alignment in a corresponding sub-section, it is determined using the recorded data whether or not global alignment is performed in the corresponding section, and then global alignment is performed only when it is determined that there is no global alignment.

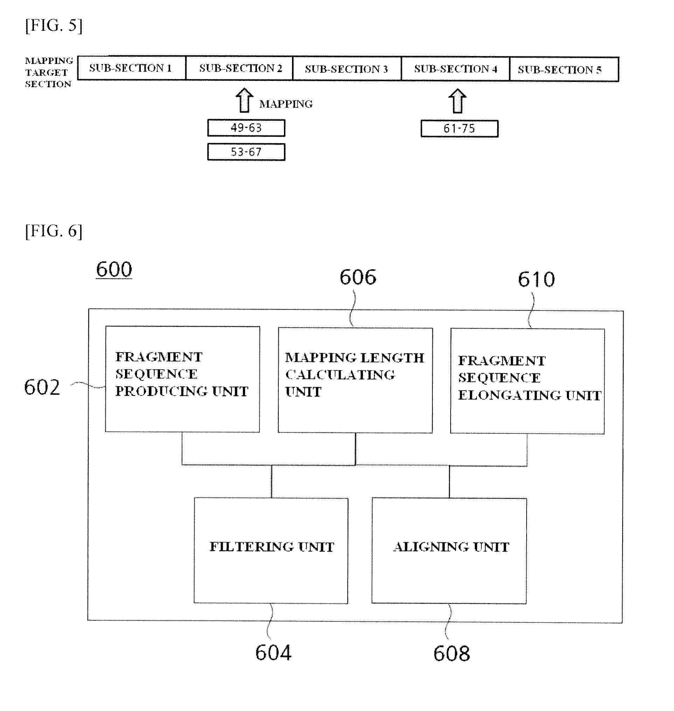

This process will be explained with reference to FIG. 5. As shown in FIG. 5, when mapping target section is divided into five sub-sections, the 49-63 and 53-67 fragment sequences out of the three final candidates are mapped to the second sub-section, and the 61-75 fragment sequence is mapped to the fourth sub-section. In this case, if global alignment with respect to the 49-63 fragment sequence is performed in the second sub-section, global alignment with respect to the 53-67 fragment, which is included in the same sub-section, is not performed regardless of the result, and vice versa. Therefore, in the exemplary embodiment shown in FIG. 5, global alignment is performed only on the combination of the 49-63 and 61-75 fragment sequences or the 53-67 and 61-75 fragment sequences. Even when global alignment is performed only in the mapping target section rather than in the entire target sequence, it takes much time. Accordingly, through the above-described process, the time to perform global alignment may be reduced.

Calculation of Histogram Cut

In the exemplary embodiment, a histogram cut may be calculated by the following method.

First, f is the size of a fragment sequence, s is the shift size in a read sequence to produce the fragment sequence, L is the length of the read sequence, e is the maxError allowed in the read sequence, and H is the histogram cut. Here, the length not influenced by errors in the read sequence (T) may be calculated using the following Equation.

$$T = L-f*e-s$$

Here, L and e are predetermined before the exemplary embodiment is performed, and thus T is determined according to f and s. That is, the performance of an algorithm is changed according to how f and s are changed.

First, the following two conditions are considered to determine H. Among these, the prerequisite is necessarily satisfied, and the additional condition is considered when possible.

-Prerequisite: Since a basic unit of mapping is a fragment sequence, even when the histogram cut is small, it necessarily has a size capable of including at least two overlapping fragment sequences. If, as shown in FIG. 2, f is 15 and s is 4, the minimum length of the two overlapping fragment sequences is 15+4=19, and thus H should be at least 19. In addition, since H is set to include at least two fragment sequences, H should be at least the same as or higher than f+s. As will be described later, since value f is at least 15, if s is at least 1, H is at least 16 (=15+1).

-Additional Condition: In an ideal circumstance, when H is T or a histogram to which a sequence having T or more is mapped is found, all mapping with respect to the given error may be found. However, as described above, when the target sequence has many overlaps, a length of a fragment sequence may be elongated according to a circumstance. Accordingly, to determine H in consideration of this, the use of a bit T-s smaller than T is favorable in terms of a mapping rate. If H is T, H is T-f*e-s. Here, when e is 1, which is the minimum value (when e = 0, the fragment sequence exactly matches a target sequence, and thus mapping is completed in the previously-described step 104), H is T-F-S. This value becomes the maximum histogram value. If L = 75 bp, f = 15 bp and s = 1, the maximum value of H is 75-15-1 = 59.

Summarizing this, H necessarily satisfies the following range.

f+s <= H <= T-(f+s)

Subsequently, f is the largest of the values satisfying the following two conditions. The prerequisite is necessarily satisfied, and the additional condition is considered when possible.

-Prerequisite: f is 15 or more. This is because the number of mapping positions in the target sequence is drastically increased when the length of the fragment sequence is 14 or less. An average appearance frequency of the fragment sequence in a human genome according to a length of the fragment sequence is shown in Table 1.

[Table 1]

| Length of Fragment Sequence | Average Appearance Frequency |
|---|---|
| 10 | 2,726.1919 |
| 11 | 681.9731 |
| 12 | 170.9185 |
| 13 | 42.7099 |
| 14 | 10.6470 |
| 15 | 2.6617 |
| 16 | 0.6654 |
| 17 | 0.1664 |

As shown in Table 1, when the length of the fragment sequence is 14 or less, the frequency per fragment sequence is 10 or more. However, it can be seen that when the length of the fragment sequence is 15, the frequency is decreased to 3 or less. That is, when the length of the fragment sequence is 15 or more, the overlapping of the fragment sequence may be drastically reduced, compared with when the length of the fragment sequence is 14 or less.

-Additional Condition: f = L/(e+2) is necessarily satisfied, which is because it is ensured that the length of T is the size of the two fragment sequences or more.

For example, if L = 100 and e = 4, f necessarily has a value of 16 or less.

Summarizing the above conditions, a method of determining f, s and H is as follows:

-After s is fixed to 4, f and H are determined.

-The largest value is determined as f within the range of $15 \leq f \leq L/(e+2)$ (however, necessarily f=15).

-H is determined using the following Equation.

The largest of the values calculated by H = L-f*e-2s and H = f+s.

(Here, H is the reference value, L is the length of a read sequence, f is the length of a fragment sequence, e is the maxError of the read sequence, and s is the shift size of each fragment sequence.)

Ex 1) If L = 75 and e = 3,

F = 15~15 and thus 15,

S = 4, and

H = 75-3*15-2*4 =22.

Ex 2) If L = 100 and e = 4,

F = 15~16 and thus 16,

S = 4, and

H = 100-4* 16-2*4 = 36-8 = 28.

Ex 3) If L = 75 and e = 4,

F = 15~12, but necessarily 15 or more and thus 15,

S= 4, and

H = 75-4*15-2*4 = 15-8 = 7, but f+s = 19,

Consequently, H = 19.

FIG. 6 is a block diagram of a system 600 for aligning a genome sequence according to an exemplary embodiment. The system 600 for aligning a genome sequence according to the exemplary embodiment is a device for performing the method of aligning a genome sequence described above, the system including a fragment sequence producing unit 602, a filtering unit 604, a mapping length calculating unit 606, an aligning unit 608, and a fragment sequence elongating unit 610.

The fragment sequence producing unit 602 produces a plurality of fragment sequences from a read sequence obtained from a genome sequencer. As described above, the fragment sequence producing unit 602 produces the fragment sequences by reading out a value of the read sequence by a predetermined size at the predetermined shift size from the first base of the read sequence.
The filtering unit 604 forms a set of candidate fragment sequences from the fragment sequences that match the target sequence. Here, the set of candidate fragment sequences includes fragment sequences having numbers of unmatched bases, after exact matching with the target sequence, equal to or less than is a predetermined number,

The mapping length calculating unit 606 divides the target sequence into a plurality of sections, and calculates a mapping position of the candidate fragment sequences per section and a total mapping length of the candidate fragment sequences per section.

The aligning unit 608 selects a section divided by the mapping length calculating unit 606 in which the calculated total mapping length is the reference value or more, and performs global alignment with respect to the read sequence in the selected section. Particularly, the aligning unit 608 performs global alignment with reference to the read sequence based on a mapping position of one of candidate fragment sequences mapped in the selected section in the target sequence.

In addition, the aligning unit 608 is configured to divide the selected section (mapping target section) into a plurality of sub-sections, determine whether or not global alignment was performed in a sub-section including a position to be globally aligned in the target sequence, and perform global alignment only when it is determined that the global alignment was not performed, thereby reducing the cycle of unnecessary global alignment.

The fragment sequence elongating unit 610 calculates the number of mapping positions of the candidate fragment sequences produced in the filtering unit 604 in each target sequence, selects a fragment sequence in which the calculated number of mapping positions is more than a predetermined value, and elongates a size of the selected fragment sequence until the number of mapping position in the target sequence is the predetermined value or less. Here, the fragment sequence elongating unit 610 performs elongation by adding a base of the read sequence corresponding to a corresponding position to the beginning or end of the selected fragment sequence.

Meanwhile, an exemplary embodiment may include a computer-readable recording medium including a program for performing the methods described herein on a computer. The computer-readable recording medium may include a program command, a local data file and a local data structure, which is used alone or in combination. The medium may be specially designed or configured for the exemplary embodiment, or already known to one of ordinary skill in the field of computer software. Examples of the computer-readable recording medium include magnetic media such as a hard disk, a floppy disk and a magnetic tape, optical recording media such as a CD-ROM and a DVD, a magnetic-optical medium such as a floppy disk, and hardware devices specially configured to store and execute program commands such as a ROM, a RAM and a flash memory. Examples of the program command may include a high-level language code capable of being run by a computer using an interpreter as well as a machine code such as one made by a compiler.

According to exemplary embodiments, a seed (fragment sequence) is selected in consideration of entire reads, not only a specific region of a read sequence during alignment of the read sequence, and thus can enhance accuracy compared with an algorithm only considering a part of a read.

In addition, as the number of repetitions in a target genome sequence per fragment sequence is limited, and a length of a seed exceeding the limited number of repetitions is elongated, the mapping accuracy can be increased and a mapping speed can be also increased.

In addition, as a specific region having high possibility of being mapped with a read in a target genome sequence is selected using a mapping histogram, and global alignment is performed only in the corresponding region, a time for global alignment can be drastically reduced.

Moreover, instead of a complicated process of finding a combination with a mapping position of fragment sequences induced from a read, as global alignment is directly performed on a fragment sequence having high possibility of forming a combination, a speed of global alignment can be further increased, and as a global alignment position is recorded not to repeatedly perform global alignment near the corresponding position, cycles of unnecessary global alignment can be reduced.

While the exemplary embodiments have been shown and described with reference to certain exemplary embodiments thereof, it will be understood by those skilled in the art that various changes in form and details may be made therein without departing from the scope of the inventive concept as defined by the appended claims.

## Claims

1. A system for aligning a genome sequence, comprising:

    a fragment sequence producing unit that produces a plurality of fragment sequences from a read sequence;
    a filtering unit that forms a set of candidate fragment sequences from the plurality of the produced fragment sequences;
    a mapping length calculating unit that divides the target sequence into a plurality of sections and that calculates a total mapping length of the candidate fragment sequences for each of said plurality of sections; and
    an aligning unit that selects one of the plurality of sections for which the calculated total mapping length is equal to or greater than a reference value and that performs a global alignment of the read sequence with respect to the selected one of the plurality of sections.

2. The system according to claim 1 further comprising a fragment sequence elongating unit that calculates a number of mapping positions that map each of the candidate fragment sequences to the target sequence, selects a fragment sequence in which the calculated number of mapping positions exceeds a predetermined value, and elongates the selected fragment sequence until the number of mapping positions that map the selected fragment sequence to the target sequence approaches the predetermined value or is less that the predetermined value.

3. The system according to claim 1 or 2, wherein the fragment sequence producing unit produces the plurality of fragment sequences by reading out values of the read sequence, in a fragment size, from the first base of the read sequence up to a shift size.

4. The system according to one of claims 1 to 3, wherein the set of candidate fragment sequences includes fragment sequences having numbers of unmatched bases, after exact matching with the target sequence, equal to or less than is a predetermined number.

5. The system according to claim 2, wherein the fragment sequence elongating unit elongates the selected fragment sequence by adding a base of the read sequence, of a corresponding position, to one of the beginning and the end of the selected fragment sequence.

6. The system according to one of claims 1 to 5, wherein the aligning unit is adapted to select one of the candidate fragment sequences mapped to the selected one of the plurality of sections, and to perform the global alignment of the read sequence with respect to the selected one of the plurality of sections at a mapping position, of the selected candidate fragment sequence, in the target sequence.

7. The system according to claim 6, wherein the aligning unit is further adapted to divide the selected one of the plurality of sections into a respective plurality of sub-sections, to determine whether global alignment was performed in a sub-section including a position to be globally aligned in the target sequence, and to perform the global alignment only when it is determined that the global alignment was not performed in the sub-section.

8. The system according to one of claims 1 to 5, wherein the aligning unit uses, as the reference value, the greater of:

$$H = L - f * e - 2s,$$

wherein H is the reference value, L is the length of a read sequence, f is the length of a current one of the plurality of fragment sequences, e is the maxError of the read sequence, and s is the shift size of each fragment sequence, and

$$H = f + s.$$

9. The system according to claim 8, wherein the aligning unit selects the reference value so as to satisfy the following equation:

$$f + s \leq H \leq T - (f + s).$$

10. The system according to one of claims 1 to 5, wherein the aligning unit uses, as the reference value H such that $16 \leq H \leq 59$.

11. A method of aligning a genome sequence to align a read sequence to a target sequence, comprising:

producing a plurality of fragment sequences from the read sequence;
forming a set of candidate fragment sequences from the plurality of the produced fragment sequences;
calculating the number of mapping positions that map each of the candidate fragment sequences to the target sequence;
selecting a fragment sequence in which the calculated number of mapping positions exceeds a predetermined number;
elongating the selected fragment sequence until the number of mapping positions that map the selected fragment sequence to the target sequence approaches the predetermined value or is less than the predetermined value;
dividing the target sequence into a plurality of sections and calculating a total mapping length of the candidate fragment sequences for each of said plurality of sections ; and
selecting one of the plurality of sections for which the calculated total mapping length is equal to or greater than a reference value, and
performing a global alignment of the read sequence with respect to the selected one of the plurality of sections.

12. The method according to claim 11 adapted to operate the system for aligning a gnome sequence according to one of claims 1 to 10.

[FIG. 1]

## 100

INPUT READ ~102

TRY EXACT MATCHING ~104

EXACT MATCH? 106 — YES → ALIGNMENT SUCCESSFUL

NO

CALCULATE mEB ~108

mEB > MAXIMUM ALLOWED ERROR? 110 — YES → ALIGNMENT FAILED

NO

PRODUCE FRAGMENT SEQUENCE ~112

FORM SET OF CANDIDATE FRAGMENT SEQUENCES ~114

PRODUCE MAPPING HISTOGRAM ~116

SELECT MAPPING SECTION AND GLOBAL ALIGNMENT ~118

NUMBER OF ERRORS > MAXIMUM ALLOWED ERROR? 120 — YES → ALIGNMENT FAILED

NO

ALIGNMENT SUCCESSFUL

[FIG. 2]

[FIG. 3]

ERROR (15)          ERROR (31)          ERROR (47)

READING SEQEUNCE (75bp)

1-15
5-19
9-23
13-27
17-31
21-35
25-39
29-43
33-47
37-51
41-55
45-59
49-63
53-67
57-71
61-75

[FIG. 4]

| TARGET SEQUENCE | h[0] = 15 | h[1] = 0 | h[2] = 23 | h[3] = 15 |
|---|---|---|---|---|

⇩

SELECTED AS MAPPING TARGET

[FIG. 5]

| MAPPING TARGET SECTION | SUB-SECTION 1 | SUB-SECTION 2 | SUB-SECTION 3 | SUB-SECTION 4 | SUB-SECTION 5 |
|---|---|---|---|---|---|

⇧ MAPPING

⇧

| 49-63 |
|---|

| 61-75 |
|---|

| 53-67 |
|---|

[FIG. 6]

600       606       610

602

| FRAGMENT SEQUENCE PRODUCING UNIT | MAPPING LENGTH CALCULATING UNIT | FRAGMENT SEQUENCE ELONGATING UNIT |
|---|---|---|

| FILTERING UNIT | ALIGNING UNIT |
|---|---|

604       608

Europäisches
Patentamt

European
Patent Office

Office européen
des brevets

# EUROPEAN SEARCH REPORT

Application Number

EP 13 15 7122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | G. G. FAUST ET AL: "YAHA: fast and flexible long-read alignment with optimal breakpoint detection", BIOINFORMATICS, vol. 28, no. 19, 1 October 2012 (2012-10-01), pages 2417-2424, XP055101444, ISSN: 1367-4803, DOI: 10.1093/bioinformatics/bts456 * page 2418, left-hand column, paragraph 2 - page 2419, left-hand column * ----- | 1-12 | INV. G06F19/22 |
| X | B. NIU ET AL: "FR-HIT, a very fast program to recruit metagenomic reads to homologous reference genomes", BIOINFORMATICS, vol. 27, no. 12, 15 June 2011 (2011-06-15) , pages 1704-1705, XP055101451, ISSN: 1367-4803, DOI: 10.1093/bioinformatics/btr252 * the whole document * ----- | 1-12 | |
| X | STEPHEN M. RUMBLE ET AL: "SHRiMP: Accurate Mapping of Short Color-space Reads", PLOS COMPUTATIONAL BIOLOGY, vol. 5, no. 5, 1 May 2009 (2009-05-01), page e1000386, XP055101454, ISSN: 1553-734X, DOI: 10.1371/journal.pcbi.1000386 * abstract * * page 2, right-hand column, paragraph 3 - page 3, left-hand column * * page 8, left-hand column, paragraph 2 - page 9, right-hand column, paragraph 1 * ----- -/-- | 1-12 | TECHNICAL FIELDS SEARCHED (IPC) G06F |

The present search report has been drawn up for all claims

| Place of search | Date of completion of the search | Examiner |
|---|---|---|
| Munich | 13 February 2014 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 2 725 509 A1

Europäisches
Patentamt

European
Patent Office

Office européen
des brevets

# EUROPEAN SEARCH REPORT

Application Number

EP 13 15 7122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D. WEESE ET AL: "RazerS--fast read mapping with sensitivity control", GENOME RESEARCH, vol. 19, no. 9, 1 September 2009 (2009-09-01), pages 1646-1654, XP055101459, ISSN: 1088-9051, DOI: 10.1101/gr.088823.108 * abstract * * page 1647, left-hand column, paragraph 4 - page 1649, left-hand column, paragraph 4 * | 1-12 | |
| A | S. M. KIELBASA ET AL: "Adaptive seeds tame genomic sequence comparison", GENOME RESEARCH, vol. 21, no. 3, 1 March 2011 (2011-03-01), pages 487-493, XP055101461, ISSN: 1088-9051, DOI: 10.1101/gr.113985.110 * abstract * * page 487, left-hand column, paragraph 3 - paragraph 4 * * page 491, left-hand column, paragraph 5 - page 492, left-hand column, paragraph 2 * | 1-12 | TECHNICAL FIELDS SEARCHED (IPC) |
| A | US 2011/270533 A1 (ZHANG ZHENG [US] ET AL) 3 November 2011 (2011-11-03) * paragraph [0077] - paragraph [0119] * | 1-12 | |

The present search report has been drawn up for all claims

| Place of search | Date of completion of the search | Examiner |
|---|---|---|
| Munich | 13 February 2014 | Kürten, Ivayla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 13 15 7122

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2014

| Patent document cited in search report | Publication date | Patent family member(s) | Publication date |
|---|---|---|---|
| US 2011270533 A1 | 03-11-2011 | US 2011270533 A1<br>WO 2011137368 A2 | 03-11-2011<br>03-11-2011 |

EPO FORM P0459

For more details about this annex : see Official Journal of the European Patent Office, No. 12/82